# EUROPEAN PATENT APPLICATION

(11) **EP 3 021 599 A1**
(43) Date of publication of application: **18.05.2016**
(21) Application number: 14192604.8
(22) Date of filing: 11.11.2014
(51) Int. Cl.: H04R 25/00, A61B 5/0476

(54) **Hearing device having several modes**

(71) Applicant: Oticon A/S, 2765 Smørum (DK)
(72) Inventor: Nielsen, Claus, DK-2765 Smørum (DK); Hietkamp, Renskje K., DK-2765 Smørum (DK); Rønne, Filip Marchman, DK-2765 Smørum (DK); Olsen, Ole Fogh, DK-2765 Smørum (DK)
(74) Representative: Mikkelsen, Jacob

(57) **Abstract**

A hearing device is disclosed. The hearing device comprises a communication unit configured to receive wireless signals transmitted from a transmitting device, which hearing device comprises an output transducer adapted to generate signals that a hearing device user is able to perceive as sound, wherein the hearing device comprises a detection member configured to detect at least one activity state of the hearing device user. A hearing aid system is also disclosed.

## Description

### Field of the disclosure

The present disclosure relates to a hearing device adapted for being applied in different states of operation. More particularly, the disclosure relates a hearing device optimised for both day and night use. The disclosure also relates to a hearing system.

### Background

Hearing aid users report a need to be able to hear during their sleep. Normally hearing aid users will take off their hearing aid when they go to bed due to the fact that most hearing aids are uncomfortable to wear in bed due to the pressure behind the ear or in the outer ear. Moreover, the ear needs ventilation to get rid of the humidity that builds up in the ear canal during the day.

Many hearing aid users need to be able to hear during night time e.g. hearing their small children or a sick spouse.

Sleep studies have revealed four different stages of sleep: the drowsy stage, the light sleep stage, the deep sleep stage and the dream stage (also referred to Rapid Eye Movement, REM stage). These cycles are repeated over and over again until the person wakes up. Analyses have shown that the duration of the REM stage becomes longer as we approach the morning. The best time to be awakened is during the REM stage.

Further, getting up in the morning is a challenge for hearing aid users having problems hearing conventional alarm clocks generating audible signals. If a person is in the deep sleep phase it is very difficult to wake up. Accordingly, many hearing impaired people have to install some sort of vibrating device under their pillow in order to wake up.

Accordingly, there is a need to provide a solution that allows for waking up a hearing aid user during the REM stage.

Moreover, it is known that unaided hearing impaired persons cannot hear sounds such as a baby or a sick family member in an adjacent room, at a distance. Existing alarms applying vibration are not satisfactory, as they do not provide specific information about the state of the alarming sound, which could be coughing, throwing play toys, singing and babbling - or crying, calling for help.

### Summary of the disclosure

According to an aspect of the disclosure, the hearing device comprises a communication unit configured to receive wireless signals transmitted from a transmitting device, which hearing device comprises an output transducer adapted to generate signals that a hearing device user is able to perceive as sound, wherein the hearing device comprises a detection member configured to detect at least one activity state of the hearing device user.

Hereby it is possible to detect the activity state of the hearing device user and to use this information to pick the most appropriate time to wake-up a hearing device user.

The communication unit may be of any suitable type configured to receive wireless signals transmitted from a transmitting device. The transmitting device may be an audio monitor formed as a transmitter unit, equipped with a microphone, adapted to be placed near to a sound source (e.g. a child).

The hearing device comprises an output transducer adapted to generate signals that a hearing device user is able to perceive as sound. The output transducer may be a loudspeaker (receiver) of a hearing aid.

The hearing device comprises a detection member configured to detect at least one activity state of the hearing device user.

The detection device may comprise one or more detection members adapted for detect one or more parameter.

According to another aspect of the disclosure, the detection member is configured to detect if the hearing device user is asleep or is awake.

This allows for applying the hearing device to wake-up the hearing device user and to change the settings of the hearing device and apply a "day mode", while the hearing device user is awake, and to apply a "night mode", while the hearing device user is asleep.

According to yet another aspect of the disclosure, the detection member comprises an accelerometer and/or a gyroscope adapted to measure the orientation and/or acceleration and/or speed and/or velocity of the hearing device.

The use of a detection member comprises an accelerometer and/or a gyroscope adapted to measure the orientation and/or acceleration and/or speed and/or velocity of the hearing device allows for a simple, smart and intuitive determination the activity state of the hearing device user.

According to a further aspect of the disclosure, the detection member comprises a number of (preferably multiple) electrodes configured to record the electrical activity (electroencephalography, ECG) along the scalp of the hearing device user.

In this manner it is possible to apply ECG parameters during the user activity process determination. The ECG parameters may be applied in combination with other parameters if desired.

According to an even further aspect of the disclosure, the hearing device comprises an earmould, a dome or an earpiece, where a number of electrodes are provide on the outer periphery of the earmould, dome or earpiece of the hearing device.

The use of earmould, a dome or an earpiece, where a number of electrodes are provide on the outer periphery of the earmould, dome or earpiece of the hearing device allows for recording the EEG activity along the scalp of the hearing device user in an easy and simple manner.

According to one aspect of the disclosure, the hearing device comprises an earmould, a dome or an earpiece, where a first electrode and a second are provide on the outer periphery of the earmould, dome or earpiece of the hearing device.

According to another aspect of the disclosure, the hearing device comprises a processor, wherein the processor is configured to determine when to wake up the hearing device user on the basis of one or more signals representing acceleration, speed, orientation or EEG activity received from the detection member.

Hereby, it is possible to determine the "best time" to wake up the hearing device user.

According to yet another aspect of the disclosure, the detection member is configured to determine if the hearing device user is lying down or is in an upright position (e.g. sitting up).

The use of a detection member is configured to determine if the hearing device user is lying down or is in an upright position (e.g. sitting up) allows for using this information during user activity determination.

According to a further aspect of the disclosure, the earing device comprises a processor (and/or a signal processing circuit) that processes and/or gains the received signals from the transmitting device on the basis of the orientation of the hearing device and/or the hearing device user.

Hereby, it is possible to take into account the orientation of the hearing device and/or the hearing device user during processing and/or gaining the received signals from the transmitting device. Accordingly, a more user specific processing and/or gaining the received signals can be achieved.

The hearing aid system comprises a hearing device and a speaker unit, wherein the speaker unit comprises a receiving unit configured to wirelessly receive setting signals from the hearing device, wherein the speaker unit is provided with a unit for carrying out a synchronisation process, in which synchronisation process sound processing settings from the hearing device are transferred.

Hereby, the speaker unit can adapt the processing settings from the hearing device in an easy and safe manner.

The processing settings may include (multi-channel) gain and/or compression and other relevant settings.

According to an aspect of the disclosure, the hearing aid system comprises an acknowledging member configured to acknowledge whether or not a specific sound is important for the hearing device user.

The use of an acknowledging member configured to acknowledge whether or not a specific sound is important for the hearing device user allows for gathering information indicative of relevance for the user.

According to an aspect of the disclosure, the acknowledging member comprises a first button for acknowledging that a specific sound is important for the hearing device user and a second button for acknowledging that a specific sound is not important for the hearing device user.

This allows for gathering information in an easy, simple a user-friendly manner.

According to another aspect of the disclosure, the speaker unit comprises a processing unit configured to modify a filtering algorithm on the basis of how the hearing device user has acknowledged whether or not a number of specific sounds are important for the hearing device user, wherein the speaker unit is configured to apply the filtering algorithm to automatically process signals.

Hereby, it is possible to conduct the most user specific filtering of data (sound processing).

The filtering algorithm may include gain and/or compression.

According to another aspect of the disclosure, the speaker unit is integrated within a table stand or a pillow or a bracelet or a sweat band.

Hereby, it is possible to provide a solution in which the speaker unit is housed in a manner that fits specific user specific requirements.

According to a further aspect of the disclosure, the hearing device system comprises a first speaker unit and a second speaker unit.

Accordingly, it is possible to provide sound signals to several users at the same time - e.g. a couple sleeping in one bed. The first speaker unit and a second speaker unit may be provided in each side of the bed.

According to a further aspect of the disclosure, the first speaker unit is configured to be used by a first user and the second speaker unit is configured to be used by a second user.

According to yet further aspect of the disclosure, the hearing device is configured to be deeply fitted primarily in the bony portion of the ear canal.

In an aspect of the disclosure, a data processing system comprising a processor adapted to execute the computer program for causing the processor to perform at least some (such as a majority or all) of the steps of the method described above and in the claims is applied.

### Brief description of the drawings

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- Fig. 1A: illustrates a sleeping hearing device user wearing a hearing device;
- Fig. 1B: illustrates a close-up view of the hearing shown in Fig. 1B) together with several measurement parameters;
- Fig. 2A: schematically illustrates a curve showing the acceleration of a hearing device versus time;
- Fig. 2B: schematically illustrates a curve showing the speed of a hearing device versus time;
- Fig. 2C: schematically illustrates a curve showing the orientation of a hearing device versus time;
- Fig. 3: illustrates a hearing aid system;
- Fig. 4A: illustrates a sleeping hearing device user wearing a hearing device;
- Fig. 4B: illustrates the hearing device user shown in Fig. 4A in an upright position;
- Fig. 5: illustrates a hearing aid system;
- Fig. 6A: illustrates a deeply fitted hearing device;
- Fig. 6B: illustrates a two types of hearing devices;
- Fig. 7: illustrates a hearing aid system;
- Fig. 8: illustrates a hearing aid system and
- Fig. 9: illustrates a hearing aid system.

### Detailed description of the figures

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in an In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant.

A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems, bracelet, sweat band or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a Smartphone or other electronic device, the Smartphone/ electronic device possibly running an application that controls functionality of the at least one hearing device.

Further, an application on a Smartphone may be used to receive and present information regarding a hearing instrument wearer, e.g. a sleeping child. This allows the user, such as a parent, to remotely monitor the child and thereby utilize a hearing aid, e.g. a deeply fitted hearing aid or perhaps a part of a cochlear implant, as a device having functions similar to that of a baby monitor. A wireless link could be used to transmit information regarding sound and/or accelerometer/acceleration, etc., back to the caretaker's app with information regarding the child. Indication of activity could be given, e.g. 'sleeping', 'awake', 'lying down' etc. A live feed of sound could be sent and presented to the caretaker.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear I m plant.

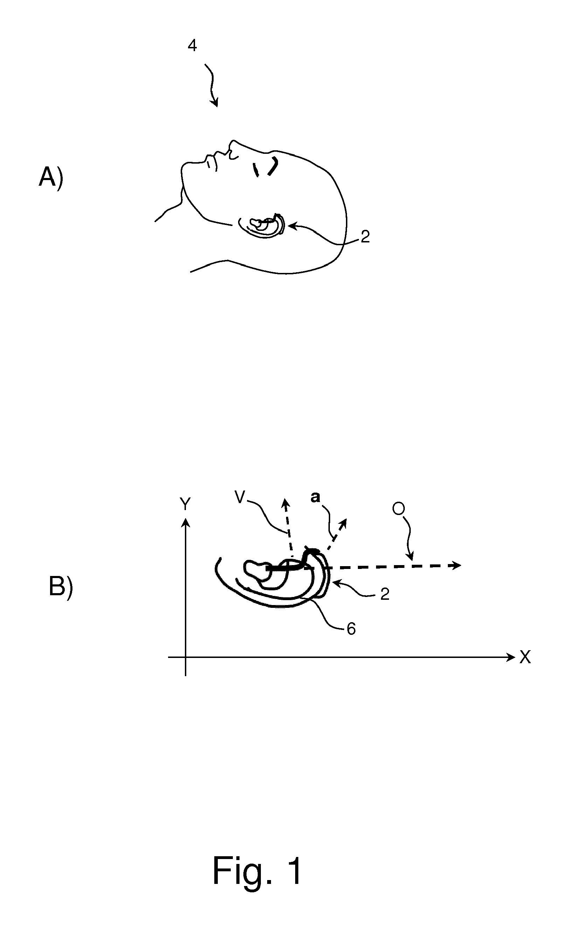

Referring now in detail to the drawings, Fig. 1A illustrates a sleeping hearing device user 4 wearing a hearing device 2 according to the disclosure.

The hearing device 2 shown in Fig. 1A is a Behind-the-Ear type hearing aid; however it is possible to apply other types of hearing aids or hearing devices, e.g. an In-the-Ear type hearing aid or In-the-Canal/ Completely-in-Canal type hearing aid.

Fig. 1B illustrates a close-up view of the hearing device 2 shown in Fig. 1A. The hearing device 2 is mounted behind the ear 6 of the hearing device user (not shown). The hearing device 2 is arranged in a Cartesian coordinate system having a horizontal axis X and a vertical axis Y.

The orientation O, the velocity V and the acceleration a vectors of the hearing device 2 are indicated in Fig. 1B and thus defined by means of the coordinate system.

The orientation O, the velocity V and the acceleration a can be detected by various units within the hearing device 2. It is possible to provide the hearing device 2 with a detection member having an accelerometer and/or a gyroscope adapted to measure the orientation O and/or acceleration a and/or the velocity V of the hearing device 2.

By detecting the orientation O and/or acceleration a and/or the velocity V of the hearing device 2, it is possible to identify the activity state of the hearing device user.

If the orientation O of the hearing device 2 is close to horizontal like indicated in Fig. 1A, it would be more than likely that the hearing device user is asleep, especially if the velocity and acceleration of the hearing device 2 is close to zero.

It may be an advantage that the hearing device 2 comprises a processor, wherein the processor is configured to receive one or more signals representing acceleration a, velocity V or orientation O of the hearing device 2 and that the processor is configured to combine several signals in order to take more than one parameter into account. The hearing device may preferably be configured to apply an algorithm that automatically processes several received signals including the orientation O and either the acceleration a or the velocity V (or both) of the hearing device 2.

The hearing device 2 is preferably equipped with a wake-up member configured to determine when to wake up the hearing device user on the basis of one or more signals representing acceleration a, speed V, orientation O or electroencephalography (ECG) activity received from a detection member of the hearing device 2.

In this manner the hearing device 2 may be applied in the night time and be used as a wake-up device.

It may be an advantage if the hearing device 2 comprises a processor configured to determine when the hearing device user has been laying down for a while. It is preferred that the hearing device 2 comprises a processor adapted to (based on a predefined criterion) automatically detect when the hearing device user is asleep and hereafter shifting the hearing device into "night time mode".

It is preferred that the hearing device 2 comprises a processor adapted to (based on a predefined criterion) automatically detect when the hearing device user is awake and hereafter shifting the hearing device into "day time mode".

The "night time mode" may involve application of a special setting with a lower gain setting than in "day time mode" in order to more particularly focusing on picking up e.g. alarm sounds or speech.

It may be an advantage that the hearing device 2 is a deeply fitted hearing device configured to be arranged primarily in the bony portion of the ear canal in which there are no sweat or cerumen glands in order to reduce the need for ventilation of the ear 6.

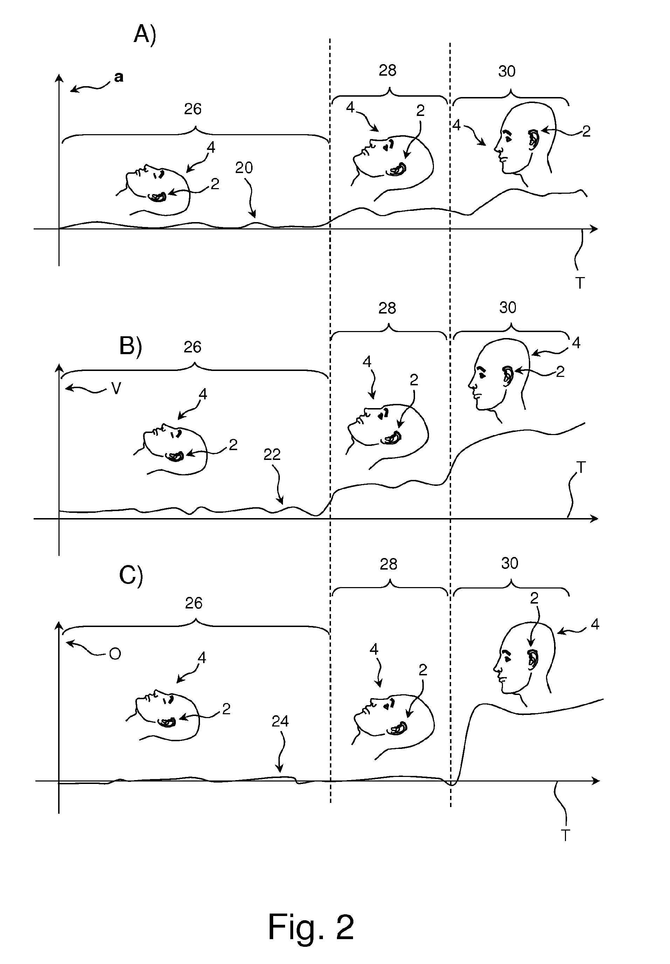

Fig. 2A illustrates a curve 20 showing the acceleration a of a hearing device 2 versus time T. The curve 20 is divided into three sections including a sleeping period 26, a waking-up period 28 and an awake period 30.

A hearing device user 4 is indicated above the curve 20 in each of the three periods 26, 28, 30. It is indicated that the hearing device user 4 is laying down in the sleeping period 26, that the hearing device user 4 is getting up in the waking-up period 28 and that the hearing device user 4 is in an upright position during the awake period 30.

The curve 20 shows that the acceleration a of the hearing device 2 is close to zero during the sleeping period 26, that the acceleration a of the hearing device 2 is slightly increased during the wake-up period 28. Moreover it can be seen that the acceleration a of the hearing device 2 is significantly higher during the awake period 30 than during both the sleeping period 26 and during the waking-up period 28.

Fig. 2B illustrates a curve 22 showing the speed (the magnitude of the velocity) V of the hearing device 2 shown in Fig. 2A versus time T. The curve 22 is dived into the same three periods: a sleeping period 26, a waking-up period 28 and an awake period 30 as indicated in Fig. 2A.

Likewise, a hearing device user 4 is indicated above the curve 22 in each of the three periods 26, 28, 30. Accordingly, it is indicated that the hearing device user 4 is laying down in the sleeping period 26, that the hearing device user 4 is getting up in the waking-up period 28 and that the hearing device user 4 is in an upright position during the awake period 30.

The curve 22 shows that the speed V of the hearing device 2 is close to zero during the sleeping period 26, that the speed V of the hearing device 2 is slightly increased during the wake-up period 28 and that the speed V of the hearing device 2 is significantly increased during the awake period 30.

Fig. 2C illustrates a curve 24 showing the orientation O of the hearing device 2 shown in Fig. 2A and in Fig. 2B versus time T. Like in the two previous figures, Fig. 2A and Fig. 2B, the curve 24 is dived into the same three periods: a sleeping period 26, a waking-up period 28 and an awake period 30. A hearing device user 4 is indicated above the curve 24 in each of the three periods 26, 28, 30.

Thus, it can be seen that the hearing device user 4 is laying down in the sleeping period 26, that the hearing device user 4 is getting up in the waking-up period 28 and that the hearing device user 4 is in an upright position during the awake period 30.

The curve 24 shows that the orientation O of the hearing device 2 is close to zero (measured relative to horizontal) during the sleeping period 26, that the orientation O of the hearing device 2 is slightly increased during the wake-up period 28 and that the orientation O of the hearing device 2 is significantly increased during the awake period 30 corresponding to the change of posture from laying during the sleeping period 26 to sitting or standing during the awake period 30.

All three parameters orientation O, velocity V and acceleration a of the hearing device 2 are candidates to estimate the activity state of the hearing device user 4. However, it may be an advantage to apply a combination of several parameters in order to increase the likelihood of identifying the activity state of the hearing device user 4 correctly.

The hearing device 2 may preferably comprise a processor configured to receive and combine several parameters e.g. including the orientation O, velocity V and acceleration a of the hearing device 2 in order to identify the activity state of the hearing device user 4 in the most optimum manner.

During the sleeping period 26 the hearing device 2 will register the orientation and movement of the head of the hearing device user 4 and thus of the orientation and movement of the hearing device 2 by means of a detection member (not shown). The processor of the hearing device 2 is then capable of conducting a sleep analysis based on the registered orientation and movement of the hearing device 2. When the normal wake-up time (which preferably is predefined) is approaching, the hearing device 2 will determine the "best time" to wake-up the hearing device user 4.

When it is time to wake-up the hearing device user 4, the hearing device 2 may initiate a fine vibration and keep on doing it until the hearing device user 4 sits up or stands up and the orientation O accordingly changes.

The hearing device 2 may comprise a detection member comprises a number of (preferably multiple) electrodes configured to record the electrical activity (electroencephalography, ECG) along the scalp of the hearing device user 4.

It may be preferred that the processor of the hearing device 2 receives EEG activity measurements from the detection member of the hearing device 2 and apply these measurements during the determination of the "best time" to wake-up the hearing device user 4.

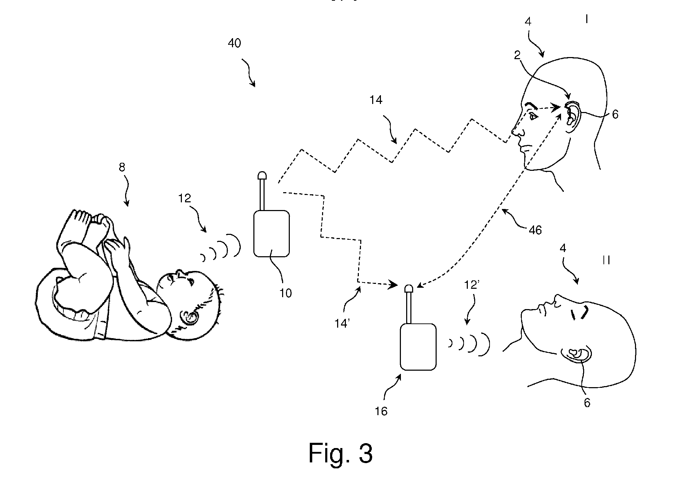

Fig. 3 illustrates a schematic view of a hearing aid system 40 according to the disclosure. The hearing aid system 40 comprises a first transmitting device 10 arranged next to a baby 8 generating an acoustic sound signal 12. The first transmitting device 10 is configured to receive the acoustic sound signal 12 and to transmit an electrical signal (on the basis of the electric field generated by the transmitting device 10) 14, 14' to a receiving device 16 comprising a speaker unit 16 adapted to generate and transmit acoustic sound 12'.

The hearing aid system 40 comprises a hearing device 2 and a speaker unit 16, wherein the speaker unit 16 comprises a receiving unit configured to wirelessly receive setting signals from the hearing device 2. The speaker unit 16 is provided with a unit for carrying out a synchronisation process, in which synchronisation process sound processing settings from the hearing device 2 are transferred.

In this manner the speaker unit 16 is capable of adapting the settings already applied by the hearing device 2. Accordingly, the speaker unit 16 will be configured to generate and transmit acoustic signals 12' that fit the hearing device user 4.

Fig. 3 shows a day scenario I as well as a night scenario II. During day time, in which the hearing device user 4 is wearing a hearing device 2 (here show as a BTE type hearing device 2 arranged behind the ear 6 of the hearing device user 2), electrical signals 14 are transmitted from the first transmitting device 10 and received by the hearing device 2.

Accordingly, the hearing device user 4 will be able to hear (by means of the receiver within the hearing device 2) an acoustic sound corresponding to the acoustic sound 12 picked up by the first transmitting device 10 and being processed in order to meet the individual requirements (defined by the hearing device settings of the hearing device 2) of the hearing device user 4.

During the night scenario II the hearing device user 4 has removed the hearing device 2 from his ear 6 and thus he is sleeping without any hearing device. Accordingly, an external device is required in order to generate the sound that is needed in order to alert the hearing device user 4 in case of e.g. a crying baby 8.

The hearing aid system 40 comprises speaker unit 16 comprising: a receiving unit configured to wirelessly receive setting signals from the hearing device 2 and a unit for carrying out a synchronisation in which sound processing settings from the hearing device 2 are transferred to the speaker unit 16. The transfer of sound processing settings from the hearing device 2 to the receiving unit of the speaker unit 16 is indicated by a dotted arrow 46.

Thus, during night time (indicate as the night scenario II) the speaker unit 16 generates and transmits acoustic signals 12' corresponding to the signals received by the first transmitting device 10. Since the speaker unit 16 has adapted the sound processing settings from the hearing device 2, the speaker unit 16 is capable of generating a sound 12' that is processed in order to meet the individual requirements of the hearing device user 4.

Accordingly, the hearing aid system 40 is capable of providing a user-optimized sound during day time (day time scenario I) as well as during the night time (night time scenario II). Moreover, in case that the hearing device settings of the hearing device 2 should change, the speaker unit 16 will automatically adapt.

The speaker unit 16 may be designed as a table stand or be integrated in a pillow or provided in another suitable manner. Regarding the speaker unit 16, although here shown within a pillow - can be incorporated in other user friendly wearables, such as a bracelet or a sweat band.

The solution proposed enables a hearing impaired person to hear the sounds, so he/she can decide how to respond. The solution proposed enables hearing impaired persons to hear their loved ones at a distance when not using hearing aids, e.g. at night.

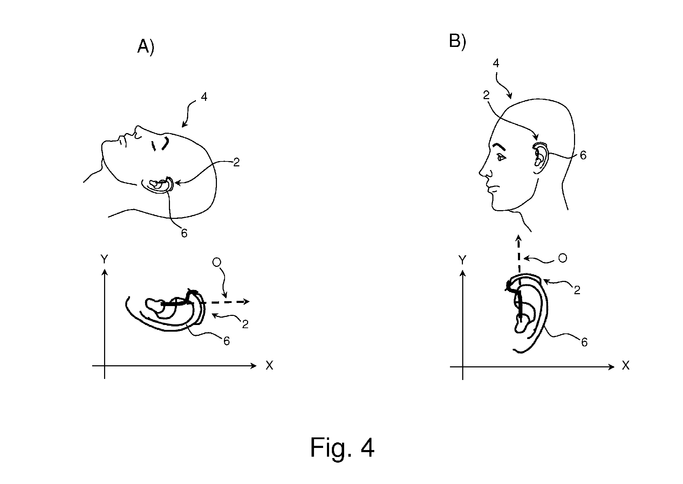

Fig. 4A illustrates a sleeping hearing device user 4 wearing a hearing device 2. The hearing device 2 is mounted behind the ear 6 of the hearing device user 4.

Below the hearing device user 4 a close-up view of the hearing device 2 is arranged in a Cartesian coordinate system provided with a horizontal axis X and a vertical axis Y. In this coordinate system the orientation O of the hearing device 2 is indicated.

The orientation O (as well as other parameters such as the velocity and the acceleration of the hearing device 2) can be detected by various units within the hearing device 2. By detecting the orientation O and optionally other parameters such as the acceleration and/or the velocity of the hearing device 2, it is possible to identify the activity state of the hearing device user 2.

As the orientation O of the hearing device 2 is close to horizontal in Fig. 4A, the hearing device 2 may determine that the hearing device user 4 is asleep.

Fig. 4B illustrates the hearing device user 4 shown in Fig. 4A in an upright position (e.g. in a sitting or standing position). A close-up view of the hearing device 2 is arranged in a Cartesian coordinate system below the hearing device user 4. The orientation O of the hearing device 2 is indicated in the coordinate system. It can be seen that the orientation O is basically vertical. Thus, a vertical orientation O indicates that the hearing device user 4 has an upright position and thus is awake.

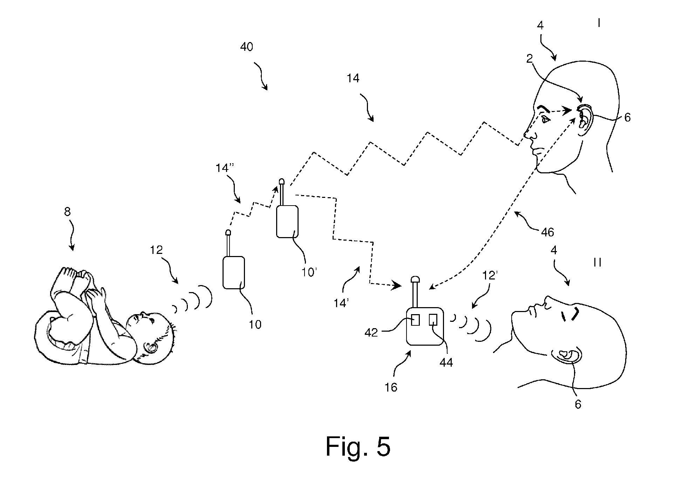

Fig. 5 schematically illustrates a hearing aid system 40. The hearing aid system 40 comprises a first transmitting device 10 arranged next to a baby 8 generating an acoustic sound signal 12. The first transmitting device 10 is configured to receive the acoustic sound signal 12 and to transmit an electrical signal 14" to a second transmitting device 10'.

The second transmitting device 10' is adapted to transmit a wireless electrical signal 14' to a receiving device. The hearing aid system 40 comprises a hearing device 2 and a speaker unit 16 comprising a receiving unit configured to wirelessly receive setting signals from the hearing device 2.

The speaker unit 16 is equipped with a unit for carrying out a synchronisation process, in which sound processing settings from the hearing device 2 are transferred to the speaker unit 16. The transfer of sound processing settings from the hearing device 2 to the receiving unit of the speaker unit 16 is indicated by a dotted arrow 46.

In this way the speaker unit 16 adapts the settings applied by the hearing device 2. Consequently, the speaker unit 16 is capable of generating and transmitting acoustic signals 12' that fit the hearing device user 4.

Since the hearing aid system 40 comprises a second transmitting device 10' the range of application of the hearing aid system 40 is larger than the hearing aid system 40 shown in Fig. 3. Although not shown, it is possibly to apply more additional transmitting devices each having a function as a repeater. Hereby, the range of application of the hearing aid system 40 may be extended even more.

Like illustrated in Fig. 3, Fig. 5 shows a day scenario I and a night scenario II. During day time, the hearing device user 4 is wearing a hearing device 2 and electrical signals 14 are transmitted from the second transmitting device 10' and received by the hearing device 2.

Hence, the hearing device user 4 will be provided with processed sound signals (corresponding to the signal 12 received by the first transmitting device 10) processed in order to meet the individual requirements (defined by the hearing device settings of the hearing device 2) of the hearing device user 4.

During the night scenario II the hearing device user 4 wears no hearing device 2. Therefore, an external device is necessary in order to generate the sound required in order to alert the hearing device user 4 in case of e.g. a crying baby 8.

During night time (indicated as the night scenario II) the speaker unit 16 generates and transmits acoustic signals 12' corresponding to the signals received by the second transmitting device 10'. Due to the fact that the speaker unit 16 has adapted the sound processing settings from the hearing device 2, the speaker unit 16 is capable of generating a sound 12' that is processed in order to meet the individual requirements of the hearing device user 4.

Therefore, the hearing aid system 40 is capable of providing a user-optimized sound during day time (day time scenario I) as well as during the night time (night time scenario II). Additionally, in case that the hearing device settings of the hearing device 2 should change, the speaker unit 16 will automatically adapt.

The hearing aid system 40 comprises an acknowledging member 42, 44 comprising a first button 42 for acknowledging that a specific sound is important for the hearing device user 4 and a second button 44 for acknowledging that a specific sound is not important for the hearing device user 4.

The acknowledging member 42, 44 is provided as buttons 42, 44 arranged on the speaker unit 16.

This means that the hearing device user may push either of these buttons 42, 44 each time a sound is registered. Accordingly, the hearing aid system 40 will adapt to the specific individual needs of the hearing device user 4. Following an initial "registration period", the hearing aid system 40 may automatically filter sounds on the basis of the previously gathered information in such a manner that the hearing device user 4 does not have to keep on conducting the described acknowledging process.

Over time the hearing device 2 will adaptively learn to react only to the important sounds, much like a spam filter works for a regular e-mail client, and will be silent in periods of silence and in periods of unimportant sounds, as e.g. babbling.

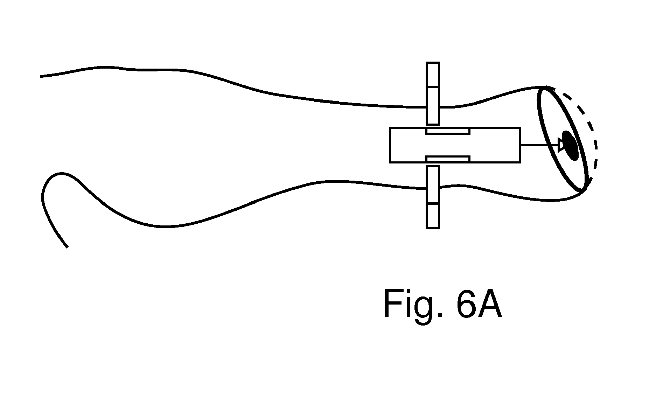

Fig. 6A illustrates a deeply fitted hearing device in an ear canal.

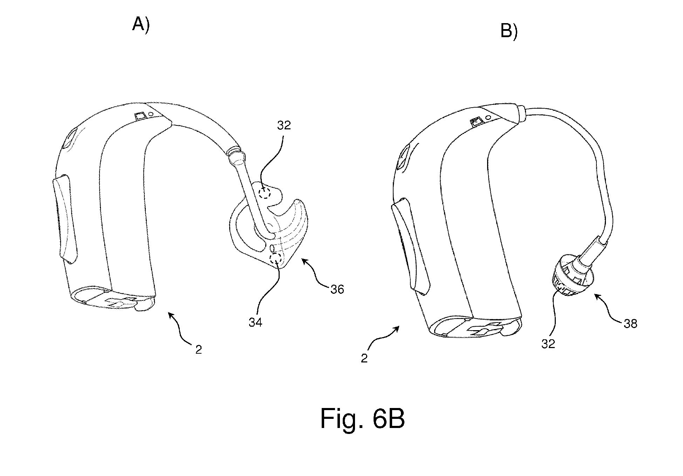

Fig. 6B illustrates a hearing device 2. The hearing device 2 is a BTE type hearing device 2 comprising an earmould 36 configured to be fitted into the ear canal of a hearing device user. The hearing device 2 comprises a detection member comprises a first electrode 32 and a second electrode 34 configured to record the electrical activity (electroencephalography, ECG) along the scalp of a hearing device user.

In practice the ECG measurement is carried out in the ear canal of the hearing device user by means of the first electrode 32 and a second electrode 34.

The electrodes 32, 34 are provided on the outer periphery of the earmould 36 of the hearing device 2.

The hearing device 2 may preferably comprise a detection member configured to detect at least one activity state of a hearing device user on the basis of ECG registrations carried out by means of the electrodes 32, 34.

A piezoelectric element may be provided in the surface of the earmould 36 and be used as a vibrator/buzzer to alarm a hearing device user e.g. when the user has to wake-up.

It is possible to detect whether a hearing device user is awake or sleeping could be by means of analysing electroencephalography, EEG potentials (e.g. REM waves) that are prominent when the hearing device user is relaxing. The EEG-signals can be picked up in the ear canal by means of the electrodes 32, 34 provided at the surface of the earmould 36.

Fig. 6B also illustrates another hearing device 2. The hearing device 2 is a BTE type hearing device 2 like the one shown in Fig. 6A. The hearing device 2 comprises dome 38 configured to be received by the ear canal of a hearing device user. The hearing device 2 comprises a detection member comprises a first electrode 32 and a second electrode (not shown) configured to record the EEG activity along the scalp of a hearing device user by means of the first electrode 32 and a second electrode. The electrodes 32 are provided on the outer periphery of the dome 38 of the hearing device 2.

The hearing device 2 may comprise a detection member configured to detect at least one activity state of a hearing device user on the basis of ECG registrations carried out by means of the electrodes 32.

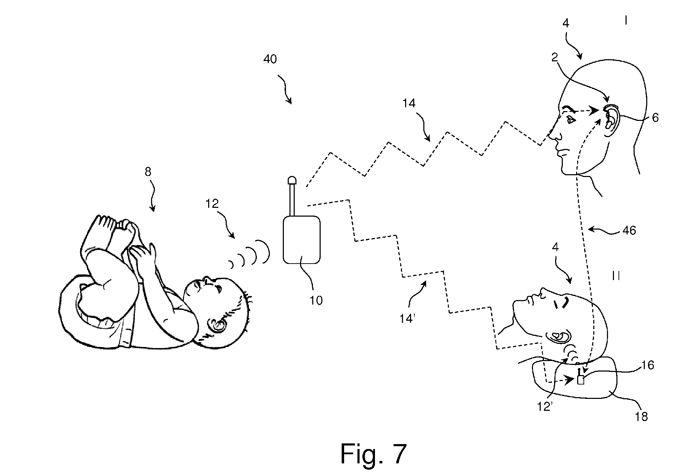

Fig. 7 illustrates a hearing aid system 40. The hearing aid system 40 basically corresponds to the one shown in Fig. 3. Unlike the hearing aid system 40 shown in Fig. 3, the hearing aid system 40 illustrated in Fig. 7 comprises a receiving device 16 integrated within a pillow 18.

The hearing aid system 40 shown in Fig. 7 comprises a first transmitting device 10 arranged next to a baby 8 generating an acoustic sound signal 12. The transmitting device 10 is adapted to receive the acoustic sound signal 12 and to transmit electrical signals 14, 14' to a receiving device 16 within the pillow 18. The receiving device 16 comprising a speaker unit 16 adapted to generate and transmit acoustic sound 12'.

The hearing aid system 40 comprises a hearing device 2 and a speaker unit 16. The speaker unit 16 comprises a receiving unit configured to wirelessly receive setting signals from the hearing device 2 and the speaker unit 16 is provided with a unit for carrying out a synchronisation process, in which synchronisation process sound processing settings from the hearing device 2 are transferred.

Accordingly, the speaker unit 16 can adapt the settings already applied by the hearing device 2, and thus, the speaker unit 16 can generate and transmit acoustic signals 12' that fit the hearing device user 4.

Fig. 7 shows a day scenario I and a night scenario II. During day time, electrical signals 14 are transmitted from the transmitting device 10 and received by the hearing device 2 in such a manner that the hearing device user 4 is capable of hearing an acoustic sound corresponding to the acoustic sound 12 picked up by the transmitting device 10. The acoustic sound 12 picked up by the transmitting device 10 are being processed in order to meet the individual requirements defined by the hearing device settings of the hearing device 2.

During the night scenario II the hearing device user 4 is sleeping without any wearing the hearing device 2. Thus, an external device is necessary in order to generate the sound that is required in order to alert the hearing device user 4 in case of e.g. a crying baby 8.

The speaker unit 16 comprising: a receiving unit configured to wirelessly receive setting signals from the hearing device 2 and a unit for carrying out a synchronisation in which sound processing settings from the hearing device 2 are transferred to the speaker unit 16. The transfer of sound processing settings from the hearing device 2 to the receiving unit of the speaker unit 16 is indicated by a dotted arrow 46.

Accordingly, during night time the speaker unit 16 within the pillow 18 generates and transmits acoustic signals 12'. Due to the fact that the speaker unit 16 has adapted the sound processing settings from the hearing device 2, the speaker unit 16 is capable of generating a sound 12' that is processed in order to meet the individual requirements of the hearing device user 4.

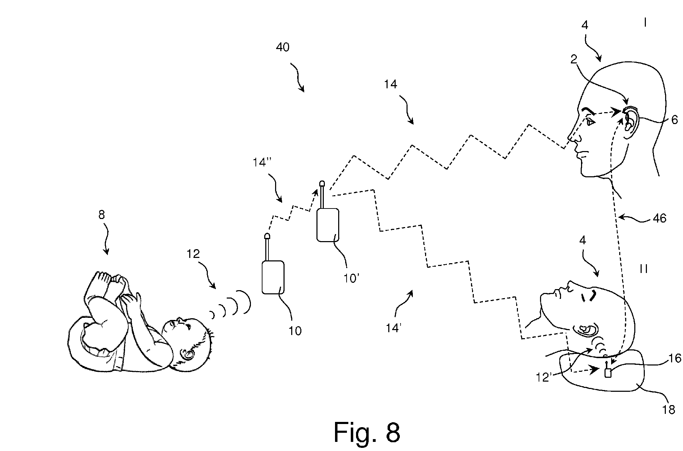

Fig. 8 illustrates a hearing aid system 40. The hearing aid system 40 basically corresponds to the one shown in Fig. 7, however, the hearing aid system 40 comprises a second transmitting device 10' configured to receive electrical signals 14" transmitted from a first transmitting device 10 arranged in a close distance to the baby 8.

The second transmitting device 10' is adapted to transmit electrical signals 14, 14' to the hearing device 2 that the hearing device user 4 is wearing or to the speaker unit 16 provided in the pillow 18, respectively.

By means of the additional transmitting device 10' it is possible to pick up sound signals 12 in a larger distance from the hearing device user 4. It would be possible to apply several additional transmitting devices (not shown) in order to increase the range of application of the hearing aid system 40.

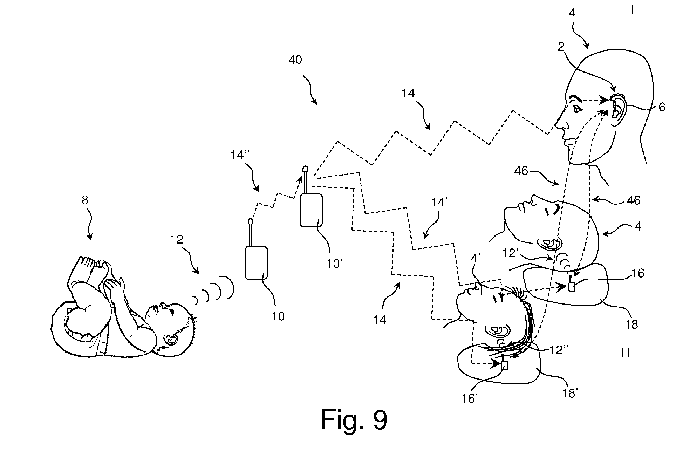

Fig. 9 illustrates a hearing aid system 40. The hearing aid system 40 essentially corresponds to the one shown in Fig. 8; however, the hearing aid system 40 both comprises a first pillow 18 provided with a speaker unit 16 and a second pillow 18' provided with a speaker unit 16'.

In Fig. 9 a first individual 4 and a second individual 4' are sleeping next to each other in the night scenario II. The hearing aid system 40 comprises a first transmitting device 10 receiving acoustic signals 12 from a baby 8 and transmitting an electric signal 14" to a second transmitting device 10'. The second transmitting device 10' transmits an electrical signal 14 directly to the hearing device 2 that the first individual 4 is wearing during day time I.

The second transmitting device 10' transmits electrical signals 14' to the first speaker unit 16 within the first pillow 18 (used by the first individual 4 during night time II) and to the second speaker unit 16' within the second pillow 18' (used by the first individual 4 during night time II), respectively.

Although only one hearing device user 4 is shown during day time I, it is possible to have more hearing device users 4, 4' receiving the electrical signal 14 from the second transmitting device 10' during day time.

It is possible to have a first speaker unit 16 equipped with a unit for carrying out a synchronisation process, in which sound processing settings from a first hearing device 2 are transferred to the first speaker unit 16 and to have a second speaker unit 16' equipped with a unit for carrying out a synchronisation process, in which sound processing settings from a second hearing device that a second hearing device user is wearing are transferred to the second speaker unit 16'. In this manner it would be possible to have several user of the hearing aid system 40.

It is an advantage that the first speaker unit 16 and/or the second speaker unit 16' can be synchronised with the hearing device 2 in order to apply gain corrected for the hearing loss of the hearing device user 4.

The disclosure mates it possible for a hearing device user to 'listen in' on a loved one, to hear all sounds and thereby enabling the listener to decide on the proper intervention.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

### List of reference numerals

- 2: Hearing device
- 4, 4': Hearing device user
- 6: Ear
- 8: Baby
- 10: Transmitting device
- 12: Sound signal
- 14, 14': Wireless signal
- 16, 16': Loud speaker
- 18, 18': Pillow
- 20, 22, 24: Curve
- 26: Sleeping period
- 28: Waking-up period
- 30: Awake period
- 32, 34: Electrode
- 36: Earm ould
- 38: Dome
- 40: Hearing aid system
- 42, 44: Button
- 46: Transfer of sound processing settings
- T: Time
- X, Y: Axis
- O: Orientation
- V: Speed
- **a**: Acceleration
- I: Day scenario
- II: Night scenario

## Claims

1. A hearing device (2) comprising a communication unit configured to receive wireless signals (14, 14') transmitted from a transmitting device (10, 10'), which hearing device (2) comprises an output transducer adapted to generate signals that a hearing device user (4, 4') is able to perceive as sound, wherein the hearing device (2) comprises a detection member configured to detect at least one activity state of the hearing device user (4, 4').

2. The hearing device (2) according to claim 1, wherein the detection member is configured to detect if the hearing device user (4, 4') is asleep or is awake.

3. The hearing device (2) according to claim 2, wherein the detection member comprises an accelerometer and/or a gyroscope adapted to measure the orientation (O) and/or acceleration (a) and/or speed (V) and/or velocity of the hearing device (2).

4. The hearing device (2) according to claim 2, wherein the detection member comprises a number of (preferably multiple) electrodes (32, 34) configured to record the electrical activity (electroencephalography, ECG) along the scalp of the hearing device user (4, 4').

5. The hearing device (2) according to claim 4, wherein the hearing device (2) comprises an earmould (36), a dome (38) or an earpiece, where a number of electrodes (32, 34) are provide on the outer periphery of the earmould (36), dome (38) or earpiece of the hearing device (2).

6. A hearing device (2) according to one of the preceding claims 2-5, wherein the hearing device (2) comprises a processor, wherein the processor is configured to determine when to wake up the hearing device user (4) on the basis of one or more signals representing acceleration (a), speed (V), orientation (O) or EEG activity received from the detection member.

7. A hearing device (2) according to one of the preceding claims 2-6, wherein the detection member is configured to determine if the hearing device user (4) is lying down or is in an upright position (e.g. sitting up).

8. A hearing device (2) according to one of the preceding claims 6-7, wherein the processor processes and/or gains the received signals from the transmitting device (10, 10') on the basis of the orientation (O) of the hearing device (2) and/or the hearing device user (4, 4').

9. A hearing aid system (40) comprising a hearing device (2) and a speaker unit (16), wherein the speaker unit (16) comprises a receiving unit configured to wirelessly receive setting signals from the hearing device (2), wherein the speaker unit (16) is provided with a unit for carrying out a synchronisation process, in which synchronisation process sound processing settings from the hearing device (2) are transferred.

10. A hearing aid system (40) according to claim 9, wherein the hearing aid system (40) comprises an acknowledging member (42, 44) configured to acknowledge whether or not a specific sound is important for the hearing device user (4, 4').

11. A hearing aid system (40) according to claim 9 or claim 10, wherein the acknowledging member (42, 44) comprises a first button (42) for acknowledging that a specific sound is important for the hearing device user (4, 4') and a second button (44) for acknowledging that a specific sound is not important for the hearing device user (4, 4').

12. A hearing aid system (40) according to one of the preceding claims 9-11, wherein the speaker unit (16) comprises a processing unit configured to modify a filtering algorithm on the basis of how the hearing device user (4, 4') has acknowledged whether or not a number of specific sounds are important for the hearing device user (4, 4'), wherein the speaker unit (16) is configured to apply the filtering algorithm to automatically process signals (14, 14').

13. A hearing device system (40) according to one of the preceding claims 9-12, wherein the speaker unit (16) is integrated within a table stand or a pillow (18) or a bracelet or a sweat band.

14. A hearing aid system (40) according to one of the preceding claims 9-13, wherein the hearing device system (40) comprises a first speaker unit (16) and a second speaker unit (16').

15. A hearing device system (40) according to claim 14, wherein the first speaker unit (16) is configured to be used by a first user (4) and that the second speaker unit (16') is configured to be used by a second user (4').
